Europäisches Patentamt

European Patent Office

Office européen des brevets

(11) Publication number: **0 141 135**
**A1**

# EUROPEAN PATENT APPLICATION

(21) Application number: **84110348.4**

(22) Date of filing: **30.08.84**

(51) Int. Cl.⁴: **G 01 T 1/29**
**G 01 T 1/00**

(30) Priority: **31.08.83 JP 160003/83**

(43) Date of publication of application:
**15.05.85 Bulletin 85/20**

(84) Designated Contracting States:
**DE FR GB**

(71) Applicant: **SHIMADZU CORPORATION**
**378, Ichinofunairi-cho Kawaramachi-dori Nijo Sagaru**
**Nakagyo-ku Kyoto 604(JP)**

(72) Inventor: **Hattori, Hiroyuki**
**2-1-82, Nanryo-cho**
**Uji Kyoto(JP)**

(74) Representative: **Grams, Klaus Dieter, Dipl.-Ing. et al,**
**Patentanwaltsbüro Tiedtke-Bühling-Kinne-**
**Grupe-Pellmann-Grams-Struif Bavariaring 4**
**D-8000 München 2(DE)**

(54) Emission computed tomograph.

(57) Emission computed tomograph wherein a plurality of circular arrays of radiation detectors (3A, 3B, 3C) are arranged circumferentially about and axially of a patient (P) to be examined, with a collimator ring (CR) rotatably supported and interposed between the radiation detectors and the patient, so that the radiation emitted by a radionuclide previously introduced into the patient causes some of the radiation detectors to produce corresponding outputs, from which the position of the radionuclide within the patient's body in both the circumferential and axial directions thereof is calculated thereby to reconstruct a tomographic image of the body at a desired transverse plane or slice thereof or a three-dimensional image of a desired portion thereof.

Fig. 4

Croydon Printing Company Ltd.

BACKGROUND OF THE INVENTION

This invention relates to a radiation imaging apparatus for use in nuclear medicine, and more particularly to a ring-type emission computed tomograph.

As is well known, emission computed tomography commonly referred to as ECT is a technique for obtaining an image of the distribution of radioactivity of radionuclide in a desired plane perpendicular to the axis of the body of a patient to be examined within a particular organ thereof. To obtain such an image a pharmaceutical compound labelled with the radionuclide is first given to the patient, and the gamma radiation emitted by the radionuclide that has been accumulated in the particular organ is detected from outside the body, and the data detected are processed by an electronic computer to obtain the image.

In one known ring-type emission computed tomograph such as disclosed in U.S. Patent No. 4389569, a plurality of small-sized radiation detectors are so arranged side by side as to form an annular body or ring, in which a patient lies so that the gamma radiation from the raidopharmaceutical previously given to the patient is detected by the radiation detectors. With this prior art arrangement, the data sampling intervals in the circumferential direction depend upon the intervals at which the detectors are arranged circumferentially, so that it is practically impossible to make the intervals smaller than a certain mechanical limit.

IEEE Transactions on Nuclear Science Vol. NS-27 No. 1 disclose another ring-type of emission computed tomograph which is capable of slicing the patient under examination at different transverse planes. In this apparatus a plurality of radiation

- 1 -

detectors are arranged side by side along a circle to form a
detector ring whose center coincides with the axis of the body
of the patient, and a plurality of such detector rings are
arranged side by side in the direction perpendicular to the
slice, which direction usually coincides with the axis of the
body of the patient and will be referred to as the Z direction,
so that tomographic images at axially spaced different planes
or slices are obtained simultaneously. With this arrangement,
however, each of the axially arranged detector rings funcitons
independently of the others, and the electrical outputs from
the detectors in each of the detector rings are processed
independently of the outputs from the detectors in the other
detector rings, so that images at different transverse planes
or slices are reconstructed independently of each other. Due
to the axial distance or gap that must be mechanically provided
between adjacent detector rings the data sampling intervals in
the Z direction are considerably great.

Since the above-mentioned limitation to the data sampling
intervals in the circumferential direction can be removed by
slightly rotating the detector ring, it will not materially
deteriorate the spatial resolution of the image obtained in the
transverse plane or slice of the patient's body. On the
contrary, the above-mentioned limitaiton to the data sampling
intervals in the Z direction is critical, that is, greatly
obstructive to reconstruction of a fine three-dimensional image of
a portion of a patient's body under examination from tomographic
images at different transverse sections or slices thereof. To
avoid this, the axially arranged detector rings may be slightly
moved relative to the patient under examination axially thereof to obtain data between each adjacent two of the transverse planes or slices that can be covered by the detector rings. However, it takes much time to move either of the detector rings and the patient relative to the other.

Japanese Unexamined Patent Publication No. 58-92975 discloses an emission computed tomograph of such a type that a scintillation camera is rotated about a patient's body to be examined. Since the scintillation camera inherently has a high two-dimensional spatial resolution in the detection plane, it is possible to conduct sampling of data at close intervals in both the circumferential and axial (or Z) directions thereby to form a three-dimensional image with a high spatial resolution uniformly in the X, Y and Z directions. With this type of instrument, however, since the scintillation camera must be rotated, it takes much time to collect the required data. This is a great disadvantage.

Accordingly, the primary object of this invention is to provide an emission computed tomograph for use in nuclear medicine which has a higher spatial resolution in the X, Y and Z directions than the type in which a scintillation camera is rotated.

Another object of the invention is to provide such an emission computed tomograph as aforesaid which is suitable for reconstruction of a three-dimensional image and requires little time for collection of data necessary for image reconstruction.

The invention will be described in detail with reference to the accompanying drawings.

BRIEF DESCRIPTION OF THE DRAWING

Fig. 1 is a schematic front view of one embodiment of the invention;

- 3 -

Fig. 2 is a schematic side view, partly cut away, of the apparatus of Fig. 1;

Fig. 3 is a portion of Fig. 1 for explanation of the operation of the apparatus;

Fig. 4 is a somewhat schematic side view, in vertical section, of the emission computed tomograph in one embodiment of the invention; and

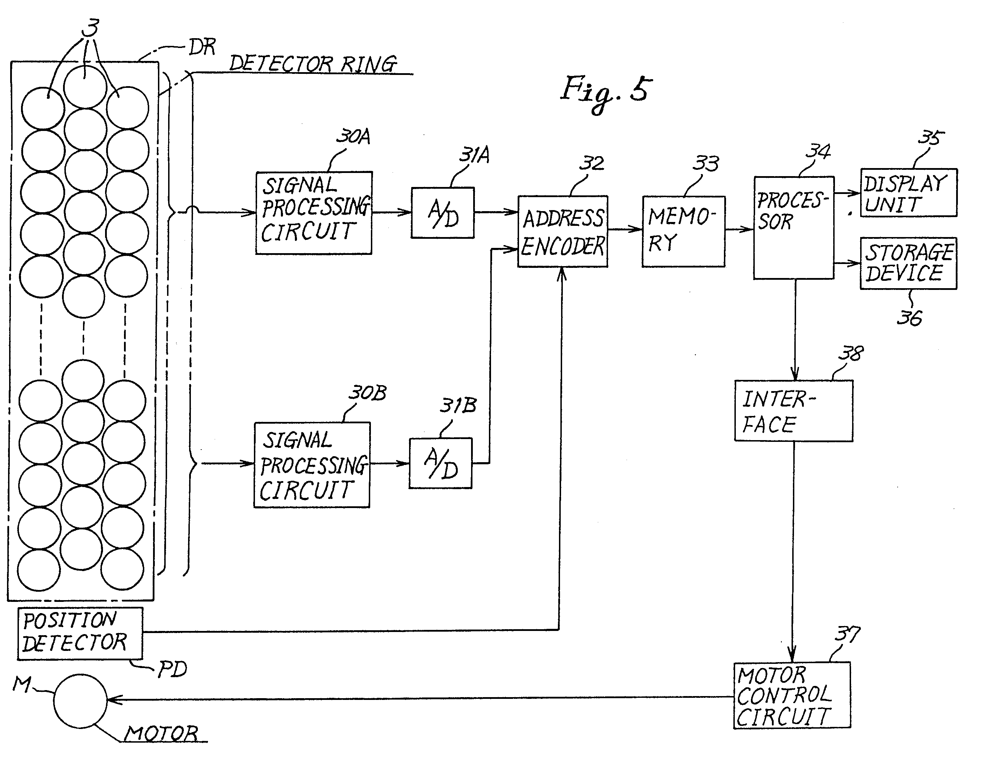

Fig. 5 is a block diagram of an image reconstruction circuit of the apparatus of the invention.

## SUMMARY OF THE INVENTION

Briefly, the imaging apparatus in one preferred embodiment of the invention is provided with a plurality of photodetectors arranged both circumferentially and axially outside and about a scintillator of a hollow cylindrical shape having a certain axial length, with a light guide being preferably interposed between the photodetectors and the scintillator. The radiation emitted by the radionuclide within the body of a patient under examination passes through a collimator arranged inside the hollow cylindrical scintillator to enter and activate the scintillator to produce scintillations. The flashes of light cause some of the photodetectors to produce corresponding output electrical signals, which are processed by an electronic computer to determine the position of occurrence of each scintillation event in both the circumferential and axial directions thereby to reconstruct tomographic or three-dimensional images.

## DESCRIPTION OF PREFERRED EMBODIMENTS

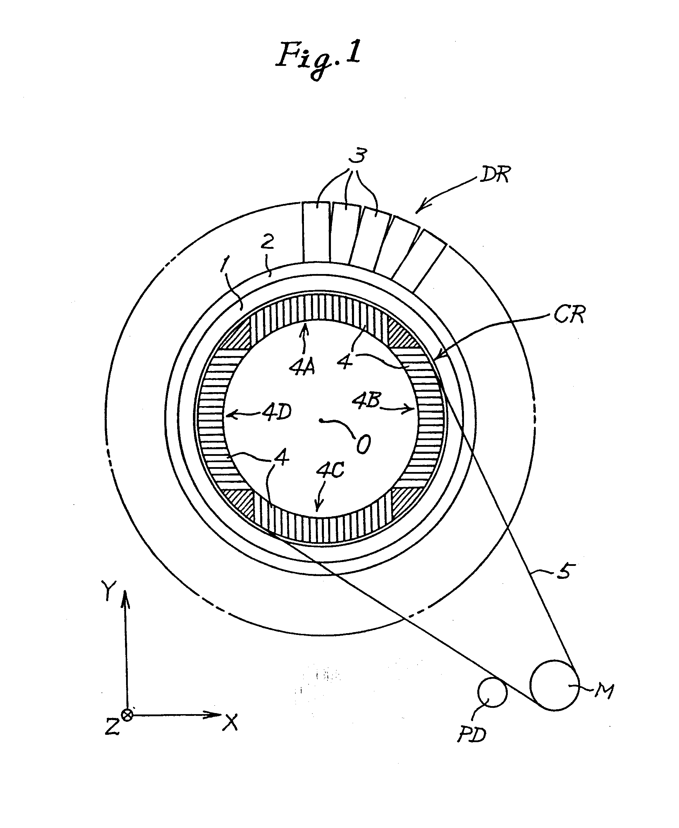

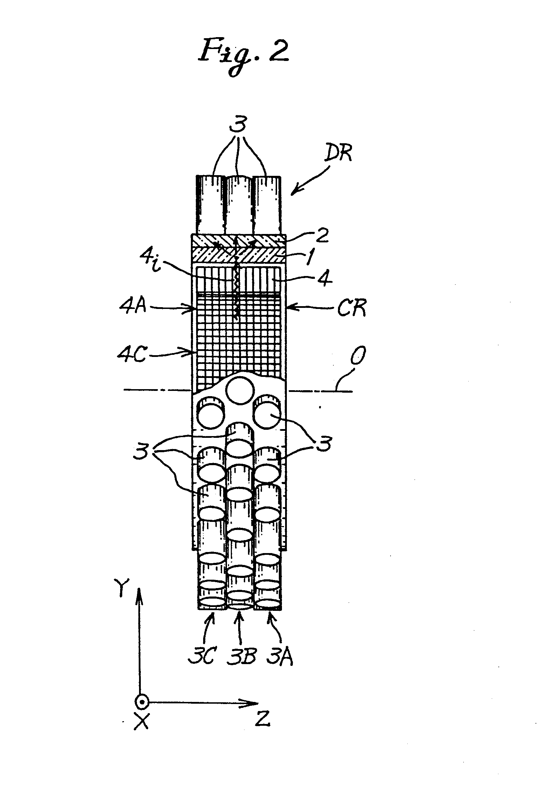

Referring now to the drawings, first to Figs. 1 through 3, there is schematically shown a scintillator 1 in the form of a hollow cylinder having an axial length, that is, extending in

- 4 -

the Z direction as shown in Fig. 2. The scintillator 1 may be formed as a single integral body from an ingot. Alternatively, it may be made by cementing several annular scintillators face to face.

A hollow cylindrical light guide 2 is provided outside the cylindrical scintillator 1, with the inner circumferential surface of the light guide 2 being in close contact with the outer circumferential surface of the scintillator 1.

Outside the cylindrical light guide 2 there is provided a detector ring DR comprising a plurality of photomultiplier tubes 3 arranged side by side with their light incident end surfaces being in contact with the outer circumferential surface of the annular light guide 2.

As shown in Fig. 2, the photomultiplier tubes 3 are arranged circumferentially as well as axially so that there are three circular arrays 3A, 3B and 3C of photomultiplier tubes arranged side by side axially of the light guide 2. If desired, there may be provided two or more than three circular arrays of photodetectors.

Inside the cylindrical scintillator 1 and concentrically therewith there is provided a collimator CR in the form of a ring or hollow cylinder which is rotatable about a common axis 0 by a suitable drive schematically shown in Fig. 1 as a belt 5 and a motor M, with a position detector PD for detecting the rotational position of the collimator ring CR.

The collimator ring CR comprises a plurality of slits 4 schematically shown in the figures. The slits 4 are arranged circumferentially of the collimator ring CR as shown in Fig. 1 and axially or in the Z direction thereof as shown in Fig. 2.

In the illustrated embodiment, as shown in Fig. 1 the collimator ring CR comprises four arcuate sections 4A, 4B, 4C and 4D  90° spaced apart from each other, in each of which the slits 4 are arranged so as to be directed toward the object positioned in the collimator ring for examination and extend in parallel with each other.  The slits in the collimator sections 4A and 4C extend in the direction perpendicular to the direction in which the slits in the sections 4B and 4D extend.  The illustrated collimator is only one of the various types of collimators that can be used in the apparatus of this invention.

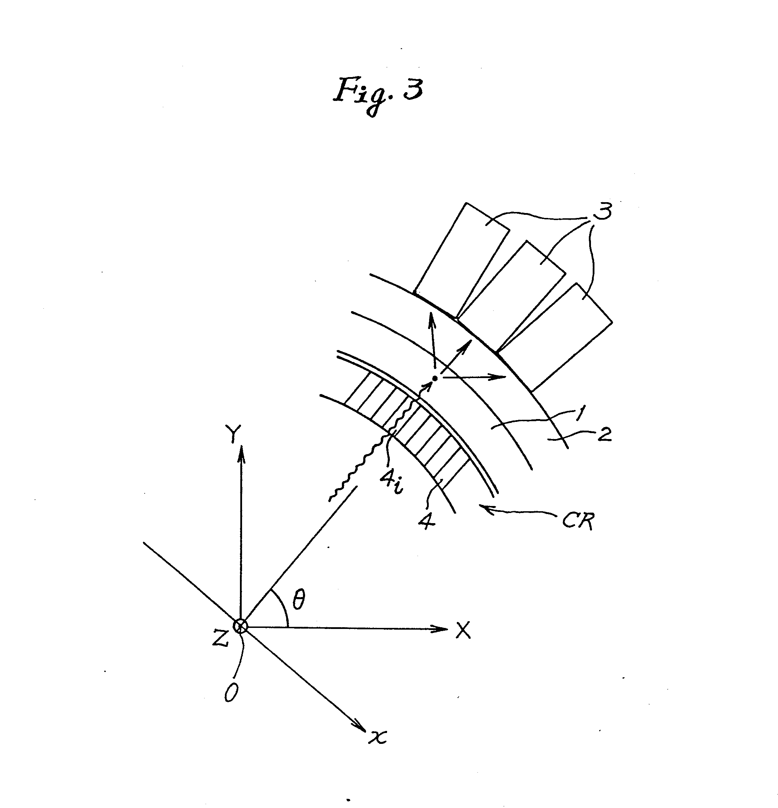

When the gamma radiation emitted by the radionuclide within the patient under examination passes through the collimator ring CR to enter the scintillator 1, flashes of light are produced within the scintillator as schematically shown in Figs. 2 and 3. The light produced is detected through the light guide 2 by those of the photomultiplier tubes 3 which are adjacent the site of the scintillation event, and  the photomultiplier tubes produce corresponding electrical outputs.  The nearer to the position of occurrence of the scintillation event the photomultiplier tube is, the greater amount of light enters the photomultiplier tube and the greater the output thereof becomes.

By applying the outputs from the photomultiplier tubes to an appropriate position calculating circuit such as for example a resistor matrix circuit used in a conventional scintillation camera of the Anger type it is possible to calculate and determine the position of occurrence of each scintillation event in both the axial (or Z) and circumferential directions.

The posiiton in the circumferential direction can be translated into a corresponding position in the direction $\underline{x}$

- 6 -

perpendicular to the direction of the slit 4i of the collimator which makes an angle θ with the reference or X axis and through which the gamma radiation passes, whereupon the position of the radiation source or radionuclide that has emitted the radiation as viewed from the direction of the angle θ relative to the X axis, that is, the position of the radionuclide in both the x and Z directions has been determined. Thus a projection image of the intensity distribution of the radionuclide on the x-Z plane has been obtained. This image is equivalent to that which would be obtained by a scintillation camera set at the angle θ toward the patient, so that substantially the same degree of spatial resolution in the x and Z directions as with conventional scintillation cameras can be attained, with the spatial resolution of the image depending upon that of the collimator CR.

In the illustrated embodiment which employs the four-direction collimator, four such projection images taken from the four directions spaced 90° apart from each other can be obtained simultaneously. The four-direction collimator may be replaced by any other multi-direction collimator. A three-direction collimator widens the field of view while an eight- or ten-direction collimator increases the overall sensitivity.

The collimator ring CR is rotated about its axis 0 to change the angle θ incrementally or continuously so as to collect data at each incremental angle or continuously in the same manner as mentioned above until the collimator is rotated through 90°, that is, one-fourth of one rotation, whereupon the projection data which are necessary to reconstruct the tomographic images have been obtained in each of a plurality of planes or slices parallel to the X-Y plane, with the distance or space between

- 7 -

each adjacent two of the slices being defined by the spatial resolution in the Z direction.

The projection data obtained in the different directions are processed by an electronic computer in accordance with an algorithm for image reconstruction such as the convolution and back projection methods as in the known emission computed tomograph to obtain an image of the distribution of radionuclide in the parallel planes or slices. When the two-dimensional images of the radionuclide distribution in the parallel planes or slices spaced in the Z direction are combined, a three-dimensional image of the distribution of radionuclide in the X, Y and Z directions is obtained. The three-dimensional image can be used for diagnosis as it is. The three-dimensional image or data may also be processed so as to obtain a tomographic image at a desired sectional plane of the patient's body under examination.

In the illustrated embodiment, for collection of projection data only the collimator ring CR is rotated while the detector ring is fixed. If desired, the position of the collimator ring CR relative to the scintillator 1, the light guide 2 and the detector ring 3 may be kept fixed, so that they can be rotated as a single structure about a patient to be examined for collection of projection data.

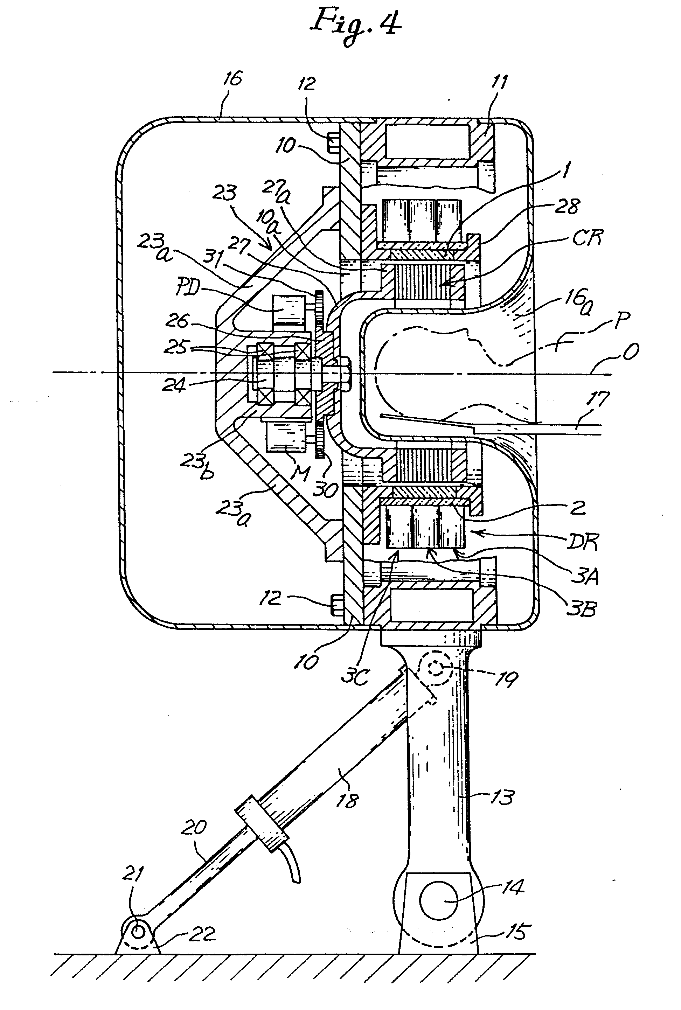

Fig. 4 shows a concrete arrangement of an imaging apparatus constructed in accordance with the invention, There is shown a stationary annular supporting plate 10 having a central opening 10a. The plate 10 is held vertically and fixed at its periphery to an annular frame 11 by means of bolts 12. The annular frame 11 is in turn fixed to and supported by a post 13 pivotally connected as at 14 to a fixed block 15. The annular

- 8 -

frame 11 forms a portion of a gantry 16 which encloses the above-mentioned supporting plate 10 and the other parts and members of the apparatus to be described hereinafter. The gantry 16 is formed in one end face thereof with an inwardly projecitng recess 16a, in which one end of a table 17 on which a patient P lies is positioned removably therefrom.

A linear motor 18 has its one end pivotally connected as at 19 to the upper end of the support post 13 and the projecting end of its actuator 20 pivotally connected as at 21 to a fixed block 22. As is easily seen, by operating the motor 18 it is possible to tilt or incline the support port 13 and the whole apparatus thereon for the purpose to be described later.

Inside the gantry 16 a spider 23 has its arms 23a fixed to one side of the supporting plate 10 and is provided with a central hub 23b, in which a spindle 24 is rotatably supported through a pair of bearings 25. A gear 26 and a bowl 27 are fixed to the spindle 24 for simultaneous rotation therewith. The bowl 27 has a flange 27a, to which a collimator ring CR is fixed for simultaneous rotation therewith. The bowl 27 is disposed coaxially within the central opening 10a of the supporting plate 10, and that portion of the end wall of the gantry 16 which defines the inwardly projecitng recess 16a is disposed coaxially within the bowl 27 and the collimator ring CR thereon.

A hollow cylindrical supporting members 28 is fixed to the side of the supporting plate 10 opposite the side thereof to which the spider 23 is fixed, so that the member 28 is axially aligned with the central opening 10a of the supporting plate 10.

A scintillator 1 in the form of a hollow cylinder is supported by the supporting member 28 so that the scintillator exteriorly encircles the collimator ring CR in radial alignment therewith.

A light guide 2 in the form of a hollow cylinder exteriorly encircles the cylindrical scintillator 1 in close optical contact therewith, and a detector ring DR comprising three circular arrays 3A, 3B, 3C of photomultiplier tubes exteriorly encircles the cylindrical light guide 2.

A motor M is fixed to the central hub 23b of the spider 23 and is provided with an output gear 30 which meshes with the gear 26 on the spindle 24.

A position detector PD is fixed to the hub 23b and is provided with an input gear 31 which meshes with the gear 26.

As the motor M is rotated, the bowl 27 and the collimator ring CR thereon are rotated through the connection of the gears 30 and 26, so that the gamma rays from inside the head of a patient P to be examined are detected in all directions around the head. The gamma rays pass through the collimator slits 4 into the scintillator 1, which produces scintillations to enter the photomultiplier tubes 3 through the light guide 2.

As the bowl 27 is rotated, the position detector PD detects the angular position of the collimator ring CR.

The outputs from the photomultiplier tubes 3 are applied to signal processing circuits 30A and 30B, one of which, say, the circuit 30A calculates the position of each scintillation event in the Z direction while the other circuit 30B calculates the position thereof in the circumferential direction (or in the $x$ direction). The outputs from the circuits 30A and 30B are applied through A/D converters 31A and 31B, resepctively, to an address encorder 32, to which the data from the position

- 10 -

detector PD concerning the angular position of the collimator ring CR are also applied. The output from the address encorder 32 is applied to a memory 33, which stores all the data collected during a quarter of one rotation of the collimator ring CR, provided that the collimator ring is of the type shown in Fig. 1.

A processor 34 reads out the data in the memory 33 and processes them so as to reconstruct a tomographic image at a required transaxial or oblique plane or a three-dimensional image of the object, which is displayed on a display unit 35 and/or recorded in a storage device 36.

The motor M which rotates the collimator ring CR is controlled by a control circuit 37 which receives instructions from the processor 34 through an interface 38.

In the illustrated upright position of the gantry 16 and the collimator ring CR therein, the image obtained is at a transverse plane perpendicular to the axis of the body of the patient P. By operating the motor 18 to incline or tilt the gantry 18 and the collimator ring CR from the illustrated vertical position to either side it is possible to obtain a tomographic image at a section oblique to the axis 0 of the patient's body.

The illustrated collimator may be replaced by any other suitable collimator of different characteristics. Instead of the photomultiplier tubes any other type of photodetectors such as a semiconductor type of photodetector may also be used.

The collimator-scintillator system of the invention can be placed close to the head or body of a patient to be examined since the system has no moving parts exposed to cause injury to the patient. The system has a higher spatial resolution than the conventional scintillation camera of the rotary type. With the system of the invention a three-dimensional image having a high spatial resolution in each of the X, Y and Z directions can be constructed. Since it is possible to obtain images simultaneously in different directions, the time required for collection of data for image reconstruction can be shortened with resulting improvement in the overall sensitivity.

CLAIMS

What I claim is:

1. An emission computed tomograph comprising: means for supporting an object to be examined at a predetermined position, said object having an axis and having taken therein a radionuclide that emits gamma radiation; radiation detecting means comprising a plurality of circular arrays of radiation detectors arranged side by side axially of said object; means for supporting said circular arrays of radiation detectors circumferentially about said object; a collimator ring arranged between said object and said radiation detecting means concentrically with said axis and comprising a plurality of slits for directing said radiation emitted from said object to said radiation detecting means; means for supporting said collimator ring rotatably about said axis; and means responsive to the outputs of said radiation detecting means to determine the position of the source of said radiation in both the circumferential and axial directions.

2. The apparatus of claim 1, wherein said radiation detectors are semiconductor radiation detectors.

3. The apparatus of claim 1, wherein said radiation detectors are photodetectors, and said radiation detecting means further includes a scintillator ring disposed between said collimator ring and said circular arrays of photodetectors, and a light guide disposed between said scintillator ring and said circular arrays of photodetectors.

4. The apparatus of claim 3, wherein said photodetectors are photomultiplier tubes.

5. The apparatus of claim 3, wherein said photodetectors are of a semiconductor type.

- 13 -

# Fig.1

# Fig. 2

*Fig. 3*

# Fig. 4

0141135

*Fig. 5*

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,Y | US-A-4 095 107 (GENNA & PANG) <br> * abstract; column 6, lines 3-13 and line 55 - column 8, line 16; figures 3-7 * | 1,3,4 | G 01 T 1/29 |
| Y | GB-A-2 005 405 (MACHLETT LABORATORIES INC.) <br> * abstract; page 2, lines 5-96; figures 1-5 * | 1 | |
| A | | 2,5 | |
| A | IEEE TRANSACTIONS ON NUCLEAR SCIENCE, vol. NS-29, no. 1, February 1982, pages 558-562, IEEE, New York, US; S. GENNA et al.: "Digital scintigraphy: concepts and designs" <br> * figure 8 * | 1,4 | TECHNICAL FIELDS SEARCHED (Int. Cl.4) <br><br> G 01 T |
| A | IEEE TRANSACTIONS ON NUCLEAR SCIENCE, vol. NS-28, no. 1, February 1981, pages 81-89, IEEE, New York, US; S.E. DERENZO et al.: "Imaging properties of a positron tomograph with 280 BGO crystals" <br> * figures 1,2 * | 1,3,4 | |

The present search report has been drawn up for all claims

| Place of search | Date of completion of the search | Examiner |
|---|---|---|
| THE HAGUE | 30-01-1985 | DATTA S. |